# EUROPEAN PATENT APPLICATION

(11) **EP 1 698 898 A2**
(43) Date of publication of application: **06.09.2006**
(21) Application number: 05386021.9
(22) Date of filing: 20.09.2005
(51) Int. Cl.: G01N 33/53, C07K 14/78

(54) **Prognostic, diagnostic and therapeutic significance in detecting and using TIN-antigen (Tubulo-Interstitial nephritis antigen) and its segments**

(30) Priority: 21.09.2004 GR 2004100378
(71) Applicant: Foundation of Biomedical Research of the Academy of Athens, 115 27 Athens (GR); Charonis, Aristidis, 154 51 Neo Psyhiko Attikis (GR); Tsilibary, Effie-Photini, 154 51 Neo Psyhiko Attikis (GR); Vlahakos, Demetrios, 154 51 Neo Psyhiko Attikis (GR)
(72) Inventor: Charonis, Aristidis, 154 51 Neo Psyhiko Attikis (GR); Tsilibary, Effie-Photini, 154 51 Neo Psyhiko Attikis (GR); Vlahakos, Demetrios, 154 51 Neo Psyhiko Attikis (GR)
(74) Representative: Tefou-Fotea, Eleni

(57) **Abstract**

TIN antigen (the acronym is derived from Tubulo-Interstitial Nephritis) is a novel macromolecular component of basement membranes. It is expressed almost exclusively in the basement membrane of the proximal tubule of the kidney. This specific expression pattern led to the hypothesis that it can be used as a marker in diseases that affect the renal parenchyma in this area. More specifically, in cases of diabetic nephropathy and acute tubular necrosis it can provide information contributing to early prognosis and diagnosis and to the follow up of the disease. Preliminary results are presented that support the hypothesis that the levels of TIN-ag in urine samples of patients with acute tubular necrosis or diabetes may be different compared to samples from healthy individuals.

### Annex to the application documents - subsequently filed sequences listing

SEQUENCE LISTING

<110> Foundation for Biomedical Research of the Academy of Athens
Charonis, Aristidis S.
Tsilibary, Effie-Photini C.
Vlahakos, Demetrios C.

<120> Prognostic, diagnostic and therapeutic significance in detecting
and using TIN antigen (Tubulo-Interstitial Nephritis antigen) and
its segments

<130> GR/21.09.04/ GRA 2004100378

<140> 05386021.9-
<141> 2005-09-20

<160> 1

<170> PatentIn version 3.3

<210> 1
<211> 476
<212> PRT
<213> Homo sapiens

<220>
<221> 120-135 peptide TIN-ag
<222> (120)..(135) CFKDGQHYEEGSVIKE
<223> This 16 amino acid sequence defines a domain unique to TIN-ag,
absent from other TIN-ag related proteins. This domain was used
as an antigen in order to generate polyclonal antibodies, used in
the assay for TIN-ag detection in the urine.

<300>
<301> B.Zhou, TR Nelson, C Kashtan, B. Gleason, AF Michael, M Vlassi
and AS Charonis
<302> Identification of two alternatively splicedforms of Human
Tubulointerstitial Nephritis Antigen (TIN-Ag)
<303> Journal of the American Society of Nephrology
<304> 11
<306> 658-668
<307> 2000
<308> GenBank/EMBL AF195116
<309> 1998-07-08
<313> (120)..(135)

<300>
<308> GenBank/ AAF08931
<309> 2002-06-10
<313> (120)..(135)

<400> 1

Met Trp Thr Gly Tyr Lys Phe Ser Ile Phe Ser Tyr Leu Thr Thr Glu
1               5                   10                  15

Ile Trp Met Glu Lys Gln Tyr Leu Ser
            20                  25

Ala Tyr Phe Thr Arg Asn His Thr Val Leu Gln Gly Thr Arg Phe Lys
        35                  40                  45

Arg Ala Ile Phe Gln Gly Gln Tyr Cys Arg Asn Phe Gly Cys Cys Glu
    50                  55                  60

Asp Arg Asp Asp Gly Cys Val Thr Glu Phe Tyr Ala Ala Asn Ala Leu
65                  70                  75                  80

Cys Tyr Cys Asp Lys Phe Cys Asp Arg Glu Asn Ser Asp Cys Cys Pro
                85                  90                  95

Asp Tyr Lys Ser Phe Cys Arg Glu Glu Lys Glu Trp Pro Pro His Thr
            100                 105                 110

Gln Pro Trp Tyr Pro Glu Gly Cys Phe Lys Asp Gly Gln His Tyr Glu
        115                 120                 125

Glu Gly Ser Val Ile Lys Glu Asn Cys Asn Ser Cys Thr Cys Ser Gly
    130                 135                 140

Gln Gln Trp Lys Cys Ser Gln His Val Cys Leu Val Arg Pro Glu Leu
145                 150                 155                 160

Ile Glu Gln Val Asn Lys Gly Asp Tyr Gly Trp Thr Ala Gln Asn Tyr
                165                 170                 175

Ser Gln Phe Trp Gly Met Thr Leu Glu Asp Gly Phe Lys Phe Arg Leu
            180                 185                 190

Gly Thr Leu Pro Pro Ser Pro Met Leu Leu Ser Met Asn Glu Met Thr
        195                 200                 205

Ala Ser Leu Pro Ala Thr Thr Asp Leu Pro Glu Phe Phe Val Ala Ser
    210                 215                 220

Tyr Lys Trp Pro Gly Trp Thr His Gly Pro Leu Asp Gln Lys Asn Cys
225                 230                 235                 240

Ala Ala Ser Trp Ala Phe Ser Thr Ala Ser Val Ala Ala Asp Arg Ile
                245                 250                 255

Ala Ile Gln Ser Lys Gly Arg Tyr Thr Ala Asn Leu Ser Pro Gln Asn
            260                 265                 270

Leu Ile Ser Cys Cys Ala Lys Asn Arg His Gly Cys Asn Ser Gly Ser
        275                 280                 285

Ile Asp Arg Ala Trp Trp Tyr Leu Arg Lys Arg Gly Leu Val Ser His
    290                 295                 300

Ala Cys Tyr Pro Leu Phe Lys Asp Gln Asn Ala Thr Asn Asn Gly Cys
305                 310                 315                 320

Ala Met Ala Ser Arg Ser Asp Gly Arg Gly Lys Arg Asp Ala Thr Lys
                325                 330                 335

Pro Cys Pro Asn Asn Val Glu Lys Ser Asn Arg Ile Tyr Gln Cys Ser
340 345 350

Pro Pro Tyr Arg Val Ser Ser Asn Glu Thr Glu Ile Met Lys Glu Ile
355 360 365

Met Gln Asn Gly Pro Val Gln Ala Ile Met Gln Val Arg Glu Asp Phe
370 375 380

Phe His Tyr Lys Thr Gly Ile Tyr Arg His Val Thr Ser Thr Asn Lys
385 390 395 400

Glu Ser Glu Lys Tyr Arg Lys Leu Gln Thr His Ala Val Lys Leu Thr
405 410 415

Gly Trp Gly Thr Leu Arg Gly Ala Gln Gly Gln Lys Glu Lys Phe Trp
420 425 430

Ile Ala Ala Asn Phe Trp Gly Lys Ser Trp Gly Glu Asn Gly Tyr Phe
435 440 445

Arg Ile Leu Arg Gly Val Asn Glu Ser Asp Ile Glu Lys Leu Val Ile
450 455 460

Ala Ala Trp Gly Gln Leu Thr Ser Ser Asp Glu Pro
465 470 475

## Description

### Technical Field of the Invention

### Basement membrane and its functional significance

The basement membrane constitutes a special differentiation of the extracellular matrix. It is present under the basal surface of cells that exhibit polarity (such as epithelial cells and endothelial cells) and fully surrounds certain other cell types (such as muscle cells and fat cells) (Vracko et al, Am. J. Pathol. 77:314, 1974). Several studies have established that basement membranes have major functions, in the context of the extracellular matrix per se and in conjuction with the cells with which they are in contact (Timpl R and Dziadek M, Intern,Rev.Exper.Pathol. 29:1, 1986).

The functions of the basement membranes include the following:
First, they act as barriers in the permeability
   important property for compatrmentalization and h
   case of the glomerular basement membrane of the kidney, where plasma protein permeability is regulated.
Second, they form a substrate on which cells attach and spread, using specific receptors for basement membrane macromolecules, mainly of the integrin family. Changes in the strength of the interactions between cells and basement membranes are the basis for several migratory processes observed mainly during development and in the adult organism as well, both in physiological conditions (leukocyte migration, embryo implantation) and under pathological situations (cancer metastasis).
Third, their components and the molecules interacting with their components may have growth factor activity, in other words they may activate intracellular signalling pathways and modify the activity of transcription factors, ending up in transcriptional alterations leading to changes in cellular phenotype.

### Components of basement membranes

In order to better understand the structure and function of basement membranes, it is important to know the exact structure and the function(s) of the constituent macromolecules. These studies have started by the 1980's and have contributed a lot of important information.

We know that the macromolecules of the basement membranes (which as a rule are not present in other parts of the extracellular matrix), are mainly type IV collagen (a collagenous glycoprotein), two glycoproteins, laminin and entactin/nidogen and the proteoglycan perlecan (a form of heparan sulphate) (Timpl R and Dziadek M Intern,Rev.Exper.Pathol. 29:1, 1986).

Following the discovery of the major components of basement membranes, studies have focused on the interactions between these macromolecules, leading to the formation of the heteropolymeric basement membrane. It was shown that type IV collagen has the ability to self-associate and to form networks, both in vitro (Yurchenco PD,and Furthmayr H Biochemistry 23:1839, 1984) and in vivo (Yurchenco PD and Reuben GC, J.Cell Biol. 105:2559, 1987).

Similar studies have shown that laminin has the ability to form networks in vitro (Yurchenco et al, J.Biol.Chem. 260:7636, 1985) καi in vivo (Yurchenco et al, J. Cell Biol. 117:1119, 1992). It was also found that entactin/nidogen has the ability to connect these two different networks (Aumailley et al, Eur.J.Biochem. 184:241, 1989) and that the side chains of proteoglycans (either the basement membrane or the transmambrane proteoglycans could play a regulatory role in the structure of these networks (Tsilibary et al, J.Biol.Chem. 263:19112, 1988, Kouzi-Koliakos et al, J.Biol.Chem. 264:17971, 1989).

TIN-ag: what is specific about it

TIN-ag is a basement membrane macromolecule, recently discovered and not very extensively studied. The name is derived from the disease Tubulo Interstitial Nephritis (TIN). Details about this disease will be given below.

The first isolation of TIN-ag was performed from rabbit kidneys, because they have an excellent extractability of their basement membranes. Initial analysis has shown that it is a glycoprotein, not containing any collagenous parts, and not similar to any of the so far known basement membrane components (Butkowski et al, J.Biol.Chem. 265:21091, 1990). Localization studies have revealed that this macromolecule is expressed mainly in the basement membrane underlying the renal proximal tubule epithelium and to a lesser extent in the basement membrane of the intestinal epithelium (Butkowski et al, Kidney Intern. 40:838, 1991).

These results underline two very important properties of this new basement membrane

which are
expression pattern, therefore, it is not a component necessary for the structural requirements of basement membranes in general and second, its expression in the proximal tubule and the intestinal epithelium is remarkable, since these are the two most prominent parts of the body where the main function is that of absorption/transfer of substances from one compartment to the other. TIN-ag must therefore be an element with specific functional significance and the result of a local differentiation of basement membranes.

Based on the findings mentioned above, a major part of the research effort of the group of the Inventor and his collaborators is focused on the structure and function of TIN-ag.

Initially, using the information from the amino acid sequence from peptides of TIN-ag and cDNA libraries from rabbit kidney, TIN-ag was cloned in the rabbit (Nelson et al, J.Biol.Chem. 270:16265, 1995). Next, based on the information from this work, human TIN-ag was cloned, by using cDNA libraries and PCR products from total RNA from the cell line HK2, derived from proximal tubule epithelium (Zhou et al, J.Amer.Soc.Nephrol. 11;658, 2000). Based on these approaches, we now know the exact amino acid sequence of TIN-ag and at the same time we know a lot of structural information that proposes possible functions. We present this information in brief:
--- TIN-ag is expressed in two isoforms; one of them (TIN1) consists of 476 amino acids and the other (TIN2) consists of 334 amino acids. The second (shorter) form is the product of alternative splicing of the common message (The sequence of TIN1 is identical with that of TIN2, but in TIN2 amino acids 103-169 and 208-301 of TIN1 are missing).
--- following the signal sequence amino acids 20-49 constitute the propeptide region, since they are not present in the mature form. The cleavage site has amino acid sequence which is a known motif for furin cleavage. Furins are a group of known endopeptidases.
--- in two regions of the molecule, amino acids 57-154 and 204-351, a lot of cysteines are clustered.
--- amino acid sequence 119-169 constitutes a EGF-like motif, usually present in extracellular matrix molecules and possibly playing a role in interactions of TIN-ag with other macromolecules and cells.
--- the carboxyl half of the sequence has a 30% homology with cathepsins, a family of cysteine proteases. This is an impressive finding, because, with the exception of acetyl-cholinesterase in the synaptic cleft, no other basement membrane macromolecule has been assigned with enzymatic activity. Preliminary results using specific substrates and zymograms suggest that TIN-ag itself may be endowed with enzymatic activity.
--- the sequence 74-103 is enriched in amino acids with carboxyl group in their side chains and has a predicted three dimensional conformation of helix-loop-helix; these two characteristics make this a motif that can possibly bind calcium ions, a situation encountered in some extracellular matrix proteins (Maurer et al, Curr.Opin.Cell Biol. 8:609, 1996). This interaction may be of significance, since TIN-ag is expressed in sites where calcium ion transport is observed.
--- there is an ATP/GTP binding motif, as well as sites where phosphorylation can be accommodated (Kanwar et al, Proc.Natl.Acad.Sci. USA 96:11323, 1999).

Beyond the above mentioned putative functions that may be dictated by the primary amino acid sequence, several other functions have been examined. These studies are based on the hypothesis that every basement membrane macromolecule most likely interacts with other basement membrane macromolecules (contributing to network formation) and with cell surface receptors (contributing to cell adhesion and spreading).

Studies of the interaction of isolated TIN-ag with type IV collagen and laminin have revealed that these reactions are in both cases specific, dose-dependent, and saturable. Scatchard analysis has shown that in both cases the affinity is at the micromolar range; one interaction site has been deduced for collagen and two for laminin (Kalfa et al, J.Biol.Chem. 269:1654, 1994). Since both laminin and type IV collagen are present in tissues in polymeric form, the role of TIN-ag in their polymerization has been studied. It was found that TIN-ag does not affect collagen polymerization but reduces in a dose-dependent manner laminin polymerization and that this action can be seen even in already formed laminin polymers (Kalfa et al, J.Biol.Chem. 269:1654, 1994).

In order to study the interaction of TIN-ag with cells, the HK2 cell line has been used; this is a human highly differentiated cell line derived from proximal tubule (Ryan et al, Kidney Intern. 45:48, 1994). Using these cells, it was established that TIN-ag is a major factor promoting cell adhesion (Chen et al, Kidney Intern. 49:153, 1995). Furthermore, specific studies using monoclonal antibodies against integrins and integrin subunits have revealed that specific receptors for TIN-ag are the integrins a3b1 and avb3. The results suggest that these two families of integrins require different interaction sites on TIN-ag (Chen et al, Kidney Intern. 49:153, 1995). Changes in cell adhesive behavior of the cells of the renal proximal tubule may be an important parameter in the case of acute renal failure (see below).

### Expression of TIN-ag

As mentioned above, one of the most remarkable features of TIN-ag is its limited expression. This is a crucial point for the invention.

Initial studies (Butkowski et al, Kidney Intern. 40:838, 1991) have shown that this protein is detected in the basement membrane under the proximal tubule epithelium and to a lesser extent in Bowman's capsule and in the basement membrane of the distal tubule in the kidney. Outside the kidney, the only tissue that showed distinct expression to a lesser extent was the intestine; minimal expression was also observed in the epidermal basement membrane and in the corneal basement membrane.

The expression of TIN-ag during embryogenesis was studied in sections of human embryonic kidney (Nelson et al, Connect.Tiss.Res. 37:53, 1998). This study revealed that TIN-ag appears first under the epithelium of the proximal tubule and Bowman's capsule, in the stage of vascular tuft. In this stage, no other basement membrane expresses TIN-ag. Furthermore, the appearance of TIN-ag is observed in an already formed basement membrane, where type IV collagen and laminin have already been expressed. This result suggests that the expression of TIN-ag is different from other basement membrane components not only in location, but also temporally. Studies with murine embryonic tissue (Kanwar et al, Proc.Natl.Acad.Sci. USA, 96:11323, 1999) have confirmed the expression of TIN-ag mainly in the renal cortex and its absence from the glomerular basement membrane. A possible role of TIN-ag in the interactions between epithelium and mesenchyme leading to organ formation and tubule formation has been proposed. This idea may be of importance in a putative role of TIN-ag in the disease of nephronophthisis .

All the above described studies have been performed at the protein level, using immunocytochemistry techniques. Studies at the mRNA level with the technique of Northern Blotting have confirmed that the main expression of TIN-ag is in the renal tissue, to a lesser extent in the intestinal epithelium and no expression has been observed in all other tissues examined. Unpublished results from the laboratory of the inventor have additionally shown that using the technique of in situ hybridization the epithelial cells of the proximal tubule are almost exclusively responsible for the expression of TIN-ag.

### The almost exclusive localization in certain regions of the kidney makes TIN-ag a very usefull marker in various pathological situations that will be mentioned below.

### These situations indude acute renal failure/acute tubular necrosis and diabetic nephropathy.

### Acute renal failure/Acute tubular necrosis

Acute renal failure is a pathological situation where there is a sudden (within hours or days) reduction of the rate of the glomerular fixation; as a result, nitrogenous substances such as urea and creatinine are retained in the circulation. In some occasions, the term "acute tubular necrosis" is used in order to describe structural and functional failure of renal tubules, so this term is considered almost synonymous to the term "acute renal failure". It should be emphasized that the term "necrosis" is not accurate, since real necrosis does not exist. As a major problem heterogeneous, local loss of epithelial cells from the proximal tubule is observed, leaving some regions of the basement membrane "naked", in other words exposed. Also, flattening of cells, distension of tubules, loss of brush border, thining of the capillary network and accumulation of material (cellular debris) inside the lumen of the tubule are observed.

The etiology of acute tubular necrosis can vary. The major cause is ischemia, due to several reasons, but also the action of several nephrotoxic agents, either exogenous (antibiotics, organic solvents, venoms, chemotherapy, antiinflammatory drugs, immunosuppressants, radiocontrast material, bacterial toxins) or endogenous (rabdomyolysis, hemolysis, ureamia) should be acknowledged. Furthermore, situations such as major surgical operations, injuries, burns, sepsis may frequently lead to acute tubular necrosis.

Acute renal failure is one of the most severe causes of increased morbidity and mortality in hospitalized patients. Morbidity of patients with post-operational renal failure ranges from 25-100%, and especially for patients in Intensive Care Units that need hemodialysis is over 50% (Novis et al. Anesth. Analg. 778:143, 1994, Zanardo et al, J.Thorac.Cardiovasc.Surg. 107:1489, 1994, Spiegel et al, Am.J.Nephol. 11:44, 1991). The application of the optimal therapeutic approach on time will certainly reduce substanfially the high morbidity rates. In order for this to happen, appropriate biological markers, sensitive and specific for the functional status of the proximal tubules are required.

### Diabetic nephropathy

Diabetes is a very common disease, known for many centuries. It is estimated that a major part of the general population, about 5%, will develop a form of diabetes. The major characteristic in diabetes is the "going through" of glucose from the circulation to the urine, that is glucosuria, as a result of increased glucose in the blood, hyperglycemia. There are two major types of diabetes, juvenile or type 1, where we have lack of insulin due to autoimmune destruction of pancreatic islets and adult onset or type 2, where we have defective action of the existing insulin. In both forms, the common characteristic is the metabolic imbalance which leads to hyperglycemia, as mentioned above (Kumar et al, Basic Pathology).

Despite advances in diabetes therapy, which have started with the isolation and administration of insulin and have evolved in the production of specific regiments, diabetes mellitus remains a major health problem, mainly due to the complications that develop. These complications are due to micro- and macro- angiopathy, and affect mainly the kidney, the retina and the peripheral nervous system (Brownlee M, Diabetes 43:836, 1994).

The present text focuses on diabetic nephropathy, despite the fact that the pathogenetic mechanism(s) for all diabetic complications may have a lot in common at the molecular level. Diabetic nephropathy is a major socioeconomic problem, since it is the main cause of end stage renal failure in the Western World (United States Renal Data System Annual Report).

Initial studies both in animal models and in human material have demonstrated that the basic structural change during diabetic nephropathy is mesangial expansion (Steffes et al, Diabetes 38:1077, 1989), which is followed by thickening of the glomerular basement membrane. Mesangium is a specific form of extracellular matrix which biochemically is very similar to the basement membrane. Mesangial expansion in diabetes is due to a combination of increased production of extracellular matrix macromolecules and reduced degradation (Ziyadeh and Goldfarb, Kidney Intern. 39:464, 1991).

Increased production of protein components and thickening has also been observed in the basement membrane of the proximal tubule of the kidney (Ziyadeh et al, Am.J.Physiol. 259:F704, 1990). Recent studies have established that the thickening of the basement membrane of the proximal tubules correlates extremely well with mesangial expansion and glomerular basement membrane thickening (Britto et al, Kidney Intern. 53:754, 1998).

Among diabetics, (despite the fact that good glucose control plays a role), 30-40% will develop diabetic nephropathy, whereas the rest will not develop this complication. It is a crucial scientific question, with no answer yet, how to discriminate the patients among the diabetics that are prone and will develop nephropathy, vis a vis those who will not. It is crucial, since this knowledge will dictate a different therapeutic approach and will minimize the risk of hypoglycemic episodes in a large number of diabetics.

The fact that TIN-ag is localized almost exclusively in the basement membrane of the renal proximal tubule and that this basement membrane may register (while thickening) the development of nephropathy from very early stages, makes TIN-ag a putative marker for early diagnosis of developing renal disease in diabetics.

### Intrerstitial nephritis and TIN-ag

Interstitial nephritis is a pathological situation characterized by tubular abnormalities, edema and cellular infiltration of the interstitium. It may lead to acute or chronic renal failure, leading to a major health problem. This situation usually coexists with glomerular abnormalities and this is the reason why it has not been studied so extensively. However, it should be stressed that when renal function is evaluated (creatinine clearance, glomerular filtration) the findings are correlated better with the tubular damage rather than the glomerular damage (Cameron Adv.Nephrol. 18:207, 1989).

In certain cases of tubulointerstitial nephritis it has been observed that antibodies are deposited on the tubular basement membrane. This deposition may be secondary to glomerular disease or other nephropathy, however, in certain cases it is due to autoantibody deposition. It is worth mentioning that autoantibodies to the renal tubular basement membrane have been instrumental in the initial isolation of TIN-ag. Further analysis of patient material has confirmed that TIN-ag is indeed the auto-antigen in these situations (Butkowski et al, Autoantibodies, Peter and Shoenfeld ed., Elsevier 1996, pp.836).

### Existing techniques and their drawbacks which can be remeded by the invention

### For Acute Tubular Necrosis

For many years, the most popular marker is serum creatinine. Serum creatinine and urea and creatinine clearance are useful biological markers for renal function, but are non specific when it comes to the localization and the assessment of the extent of the epithelial damage in the tubules (Star, Kidney Intern.54:1817, 1998).

Recently, a novel molecule has been proposed as a sensitive marker for renal damage during acute tubular necrosis, the molecule KIM-1 (Kidney Injury Molecule-1) (Han et al, Kidney Intern. 62:237, 2002). This molecule is a glycoprotein belonging to the immunoglobulin superfamily, it crosses once the cell membrane and it is expressed in regenerating renal epithelial cells, mainly in the outer part of the renal medulla and the medullar rays of the cortex, but not in the convoluted part of the proximal tubule (Ichimura et al, J. Biol. Chem. 273:4135, 1998). Urine sample examination has shown that KIM-1 is detected in large amount in patients with acute tubular necrosis due to ischemia, but minimally in patients with necrosis due to other reasons. An exception may be cases of acute tubular necrosis due to toxic substances (Ichimura et al,

Am.J.Physiol. Renal Physiol. 286:F552, 2004). A problem related to the detection of KIM-1 is the lack of information as to when exactly appears in the urine. Study of one patient has shown that KIM-1 levels reach a maximum at 48 hours (Han et al, Kidney Intern. 62:237, 2002). The value of using KIM-1 for the study of acute tubular necrosis has been questioned recently, since high levels of KIM-1 have been observed in a patient without acute tubular necrosis (Rosen and Heyman, Kidney Intern. 63:1955, 2003).

### For Diabetic Nephropathy

At present, the most reliable marker for the development of diabetic nephropathy is the extent of proteinuria (Caramori et al, Diabetes, 49:1399, 2000), estimated by the amount of albumin in the urine. However, it is clear that albuminuria is not a reliable marker of early detection of nephropathy since, on the one hand, a group of diabetics have already develop extensive renal damage before the development of microalbuminuria and on the other hand another group of diabetics with microalbuminuria come back to physiological levels of urine albumin after a certain time (Caramori et al, Diabetes, 49:1399, 2000). Clearly, new and more reliable techniques for early detection of developing nephropathy are required. These new techniques should focus either on the detection of a specific gene whose altered expression could predict predisposition (or lack of it) for nephropathy, or they should identify molecular markers in the cells or in the extracellular matrix.

It has been proposed that quantitation of the level of expression of integrins could be a predictive marker for the development of macro- and microangiopathy (Tsilibary et al, Proceedings of the 2002 European Association for the Study of Diabetes, Budapest, Hungary), but in this case measurement of integrin subunit b1 in peripheral blood monocytes does not seem to be a marker specific for nephropathy (Tsilibary et al, Proceedings of the 2002 European Association for the Study of Diabetes, Budapest, Hungary).

### Tubulointerstitial nephritis and TIN-ag

Based on the above, it is of importance to seek the exact antigenic epitope on the molecule of TIN-ag. Its exact localization would be of clinical importance both at the diagnostic and the therapeutic level. No such effort has been done so far.

Similar approaches have been successful in the case of another autoimmune disease related to a basement membrane, namely Goodpasture's syndrome (Am.J.Med.Sci. 158:864, 1919). In this syndrome where we have a combination of nephropathy and hemoragic lung disease, it has been observed that patients' sera could react in a specific manner with selected basement membranes; eventually, biochemical studies have established that the autoantigen was the a3 chain of type IV collagen (Butkowski et al,

J.Biol.Chem. 262:7874, 1987). Subsequent studies have precisely localized the antigenic epitope in the NC1 domain of a3 chain and further studies with synthetic peptides have localized the epitope in a 36 amino acid stretch close to the carboxyl end (Kalluri et al, J.Biol.Chem. 266:24018, 1991). Extracorporeal elimination of the autoantibodies using specific affinity columns contributes to a better the prognosis of the disease.

### Brief presentation of the invention as it is defined by the claims

TIN-ag is a macromolecular component of specific basement membranes, and more specifically of the basement membrane underlying the proximal tubule epithelial cells. This localization presents major diagnostic opportunities. Its detection in the urine may be used as a marker for studying the beginning and the evolution of pathological processes that affect specifically this part of the renal tissue. Such pathological processes include (but are not limited to) (a) acute tubular dysfunction due to limited perfusion, to toxic substances (endogenous and exogenous), to radiocontrast material, to drugs, etc and (b) diabetic nephropathy.

### Identification of the advantages of the invention and comparison with the drawbacks of existing techniques

In order to assess the extent of the damage of acute tubular necrosis (irrespective of the etiology) the markers in use (serum creatinine, creatinine clearance) are useful biological indicators of renal function but fail to precisely localize the damage and its entent. On the contrary, evaluation of TIN-ag in the urine may be a specific marker for the damage of the proximal tubule basement membrane.

Recent studies with the macromolecule KIM-1 offer a more specific marker, but this marker has two major differences compared to TIN-ag. First, KIM-1 is a marker for the regeneration of epithelial cells in areas where necrosis/apoptosis has developed, whereas TIN-ag is a marker for basement membrane alterations. Second, KIM-1 is detected along the whole length of the urinary tubule and mainly in the paramedullar regions, therefore it reflects changes in different parts of the nephron compared to TIN-ag. Therefore, despite the probable usefulness of KIM-1, the information provided by the detection of TIN-ag is additional and important.

For the detection of the development of diabetic complications, and more specifically of nephropathy, the so far useful marker is the level of proteinuria. However, this marker is not reliable, since several diabetic patients that have proteinuria do not develop nephropathy. If TIN-ag could be detected early in the urine of diabetics, this may become a more sensitive and useful prognostic marker.

Recent studies that propose the use of quantitative changes in the expression of integrin subunit b1 to predict the development of diabetic complications may be useful but they do not offer a specific marker for nephropathy, since they cannot discriminate between the three types of microangiopathy (nephropathy, retinopathy, neuropathy) and macroangiopathy. Therefore, TIN-ag may present a much more specific predictive marker.

Tubulointerstitial nephritis of autoimmune etiology is a rather rare clinical entity. However, the discovery of the autoantigen could definitely lead to the design of affinity columns (with the use of recombinant TIN-ag or specific peptides derived from TIN-ag), where the elimination of autoantibodies at regular time intervals will lead to a substantial improvement of the living conditions of the patients. No one has attempted such an approach so far.

### Brief description of the data shown in the figure See next section

### Detailed example of at least one use of the invention, in order to explain the application of the invention

It was mentioned above that TIN-ag has limited and specific expression in defined basement membranes, with major localization in the renal proximal tubule basement membrane. Naturally, the initial tools for its localization were a polyclonal antibody (A95) and a monoclonal antibody (A8), which are specific and have been used in our original publications.

However, recently, a novel molecule with extensive homology to TIN-ag, has been cloned. This molecule has been named TIN-ag Related Protein (Wex et al, Biochemistry, 40:1350, 2001). This protein is expressed in several tissues, with main expression in cardiac muscle and placenta. For this reason, in order to be certain that our detection system will recognize specifically TIN-ag and in order to avoid false positive results (by possible fragments of TIN-ag Related Protein in the urine), we have generated another antibody specific for TIN-ag. We have selected the sequence CFKDGQHYEEGSVIKE which corresponds to amino acids 120-135 of the human TIN-ag sequence (Zhou et al, J.Amer.Soc.Nephrol. 11:658, 2000) and for which there is no similarity to any sequence of TIN-ag Related Protein and to any other known amino acid sequence. A synthetic peptide was made and was then coupled to KLH (Keyhole Limp Hemocyanin). The comlex was used for generation of polyclonal monospecific antibodies in rabbits. The antibodies were affinity purified and were then used in further experiments with urine samples from healthy individuals and patients.

In the experiments shown below, urine samples were stored at -80°C before use. Samples were centrifuged before use at 3.000 rpm, in order to eliminate cellular elements and crystals. The supernatant was used to calculate protein concentration by the method of Bradford and next, the same amount of protein for each individual (healthy controls or patients) was analyzed by gel electrophoresis and immunobloting, using the above described anti-peptide antibody and the method of enhanced chemiluminescence, according to well known protocols. As a positive marker usefull for the quantitation of the amount of TIN-ag in the urine, a known amount of isolated TIN-ag from rabbit kidney was used in every experiment.

The results from these experiments from a part of the patients are shown below. The values have been calculated taking into account the density of the electrophoretic bands and the known amount of TIN-ag marker loaded. The values are given in arbitrary units of TIN-ag extrapolated for 1 ml of urine for each sample.

Samples from healthy individuals are shown in positions 1-15 of the diagram. The average value of the samples from healthy individuals is 334 (range from 0 to 1050).

Samples from patients with acute tubular necrosis are shown in positions 18-22 of the diagram. The average value of these samples is 6878 (range from 2750 to 13700).

For a patient that underwent open heart surgery (valve replacement) the values of TIN-ag are shown in positions 25-27 of the diagram. Position 25 shows the value before the operation (0), position 26 shows the value of TIN-ag 6 hours after the operation (3000) and position 27 shows the value of TIN-ag 24 hours after the operation (2000).

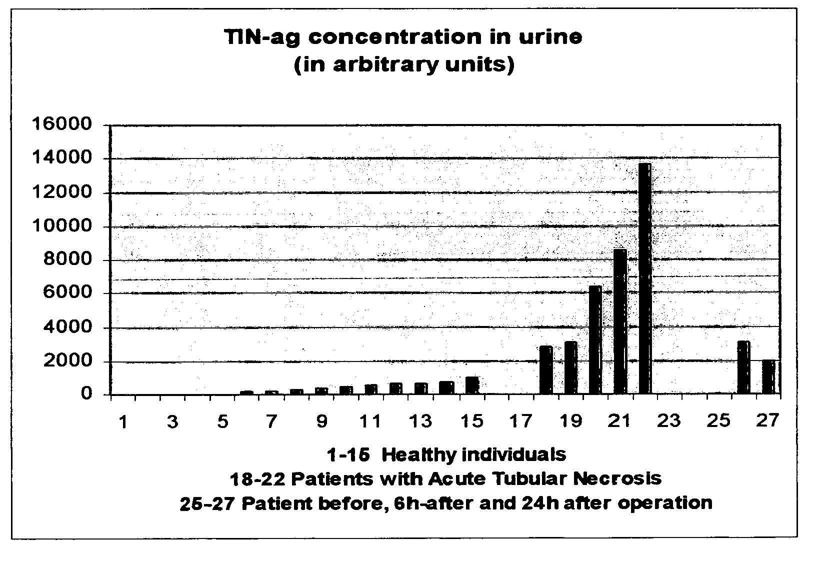

It should be mentioned that three (3) patients that suffered from nephropathy without acute tubular necrosis did not show any levels of TIN-ag in the urine.

Studies with samples from diabetics are still in very preliminary stage. Only seven (7) diabetic samples have been studied so far. Six (6) of them show a physiological range of values (0-540), but the seventh has a value of 7000. No analysis of the case histories of diabetic patients has been done so far.

## Claims

1. **Main claim**
The invention consists of:
(1) the selection of a specific sequence of TIN-ag which is not similar to other sequences of related or unrelated proteins
(2) the generation of a specific probe for this sequence
(3) the use of this probe of a specific TIN-ag sequence for prognostic and diagnostic purposes
More specifically,
(1). Based on comparisons between amino acid sequences it is concluded that the sequence described above, namely CFKDGQHYEEGSVIKE, which belongs to TIN-ag is unique and does not have any extensive identity with sequences of other known proteins.
(2). Using well known techniques of chemical synthesis and coupling with a characteristic high molecular weight protein such as KLH (Keyhole Limp Hemocyanin) we have constructed and purified with affinity chromatography a specific polyclonal monospecific anti-peptide antibody which constitutes a specific probe for this sequence.
(3). Using urine samples from patients and the above described probe, we have concluded that the use of this probe may have diagnostic value for specific pathological situations of the renal parenchyma and for the evolution of these pathological situations.
More specifically,
1. The detection of TIN-ag in the urine may be used as a marker of changes in the renal parenchyma in general and more specifically in the proximal tubule area. The study of the levels of TIN-ag could be extended to other nephropathies beyond those mentioned in this text and may lead to useful diagnostic information. More specifically, alterations of the renal parenchyma due to toxic substances (endogenous and exogenous), to reduced perfusion, to radiocontrast material given for imaging purposes, to anticancer drugs, to tissue destruction etc, could be evaluated based on the detection of TIN-ag.
2. The detection of TIN-ag may help identify patients with tubular damage and/or necrosis and the changes of TIN-ag concentration in the urine may be used as a marker of re-epithilialization. This may be applied to the above mentioned conditions but also to the follow up of the renal function of patients who have undergone major open-heart surgery and therefore need detailed follow-up. The changes in the levels of TIN-ag in the urine may be a reliable and specific marker of the evolution of the renal damage.
3. The detection of TIN-ag may possibly discriminate between diabetics with and without damage in the renal parenchyma. Up to now, the most reliable marker is considered the degree of proteinuria, but, as detailed above this marker is not so reliable. Furthermore, the therapeutic approach in diabetics with beginning renal complications may be drastically different from the approach for diabetics without complications.
4. The detection of TIN-ag in diabetics could possibly predict which individuals are prone to develop nephropathy and which ones are not. This will be an extremely useful information for their therapy. Given the fact that only 30-40% of diabetics develop nephropathy, we need to know which ones are more prone, so that we could apply only to those individuals the very strict blood glucose control. It is known that during the application of very strict glucose control severe hypoglycemic episodes are encountered. With a marker with a reliable predictive value, 60-70% of diabetics will avoid the risk of hypoglycemic episodes.
For all the above mentioned claims, the study and the detection can very easily be standarized and could be performed with easy-to-use diagnostic kits which will include the use of antibodies like the anti-peptide antibody used in the presented studies. Other similar polyclonal or monoclonal antibodies could be used with techniques like dot blot, gel electrophoresis and blotting, ELISA assays etc. Other methods using oligonucleotides (in a PCR type of reaction), and combinatory methodologies of proteomics and genomics could also be used in the future.
